# EUROPEAN PATENT APPLICATION

(11) **EP 4 446 425 A1**
(43) Date of publication of application: **16.10.2024**
(21) Application number: 22904342.7
(22) Date of filing: 09.12.2022
(51) Int. Cl.: C12Q 1/68

(54) **POST-CHEMOTHERAPY PROGNOSIS PREDICTION METHOD FOR CANINES WITH LYMPHOMA**

(30) Priority: 09.12.2021 US 202163287534 P
(71) Applicant: National University Corporation Hokkaido University, Sapporo-shi, Hokkaido 060-0808 (JP); Anicom Holdings, Inc., Shinjuku-ku Tokyo 160-0023 (JP)
(72) Inventor: YAMAZAKI, Jumpei, Sapporo-shi, Hokkaido 060-0808 (JP)
(74) Representative: Carpmaels & Ransford LLP
(86) International application number: PCT/JP2022/045551
(87) International publication number: WO 2023/106415

(57) **Abstract**

The present invention is a post-chemotherapy prognosis prediction method for a canine with lymphoma, including: a measurement step of measuring a methylation rate of one or more CpG sites selected from the group consisting of Chr4: 29559893 and Chr1: 14232692, in DNA recovered from a biological sample collected from a canine as a prediction target; and a prediction step of predicting a post-chemotherapy prognosis for a canine with lymphoma, based on the methylation rate measured in the measurement step and a preset reference value, in which the reference value is a value for differentiating between a canine having a favorable post-chemotherapy prognosis and a canine having a poor post-chemotherapy prognosis, which is set for each methylation rate of each CpG site.

## Description

### [Technical Field]

The present invention relates to a post-chemotherapy prognosis prediction method for a canine with lymphoma.

Priority is claimed on United States Patent Application, Publication No. 63/287,534, filed December 9, 2021, the content of which is incorporated herein by reference.

### [Background Art]

In veterinary clinical practice, canine lymphoma is an important disease with a high occurrence frequency. Specifically, an occurrence rate of canine lymphoma is 107/100,000 dog-years, which is 5 times or more higher than that in humans. Examples of species with a high occurrence rate include Golden Retrievers and Boxers. Canine lymphoma accounts for 24% of all tumors and > 80% of blood tumors. In terms of canine lymphoma, many canines die within 3 months in a case where treatment is not performed, but most canines can go into clinical remission because of the current development of multi-drug chemotherapy.

Examples of typical chemotherapy for canine malignant lymphoma include CHOP (chopped) therapy. The CHOP therapy is a treatment of combining three types of anticancer agents (cyclophosphamide, doxorubicin, and vincristine) with an adrenocortical hormone (prednisolone). In particular, the current most standard protocol for lymphoma called the University of Wisconsin (UW) protocol is used, and in the protocol, treatment is performed every week for the first 2 months and once every two weeks for the next 4 months, that is, treatment is performed for 6 months in total. Canine lymphoma responds well to the CHOP therapy. However, even in a case of going into clinical remission by chemotherapy, canines are in a situation where they cannot avoid recurrence and eventually die, the remission rate is > 85% and the median survival period is 1 year. In general, the recurrence occurs 3 to 4 months after the completion of treatment, and the one-year survival rate is 50%, the two-year survival rate is 20%, and the three-year survival rate is less than 10%.

Currently, as prognostic factors for canine lymphoma, those based on rough classification such as anatomical sites of occurrence, lymphocyte phenotype, clinical stages, and the like have been mainly reported. Table 1 shows those reported as prognostic factors for canine lymphoma.

**[Table 1]**

| Classification | Relapse factor | Control |
|---|---|---|
| Sex | Male | Female |
| Weight | > 18 kg | < 18 kg |
| WHO stage | V | Others |
| Sub-stage | b | a |
| Anemia | PCV < 35% | PCV > 35% |
| Anatomical sites of occurrence | Digestive organ | Multi-central type |
| Immunological classification | T cell | B cell |
| LMR (lymphocyte: monocyte ratio) | Low | High |

These prognostic factors may be prognostic factors in all the various canine lymphoma types, and there are few prognostic parameters to further subdivide each type of lymphoma. For example, prognostic factors in a homogeneous population called multi-central lymphoma have not yet been established. It is considered as a background of this that the developmental mechanism of multi-central lymphoma and the mechanism of treatment response have not been clarified.

The CpG sequence (base sequence in which C (cytosine) and G (guanine) are consecutive) has a lower appearance frequency than the simple probability, and many of the sequences are methylated. DNA methylation is modification of methyl group transfer of carbon to the 5-position of cytosine. A site where CpG is densely present is called a CpG island, and about half of mammalian genes are considered to have a CpG island in a promoter region. The methylation of cytosine in the CpG island of the promoter region causes a change in a chromatin structure of the promoter region, and thus is correlated with a decrease in the gene expression of neighboring genes. In a genome of normal cells, methylation does not occur in the CpG island in the promoter region in many cases, but these properties are dramatically changed in tumor cells, and methylation is increased in the CpG island and it is known that conversely, a reduction of methylation (a decrease in methylation rate) is often observed in a non-CpG island. That is, DNA methylation can serve as a biomarker for the onset of tumor or malignant degree thereof.

Abnormal DNA methylation has also been reported in canine lymphomas. For example, it has been reported that quantitative values of methylation amounts in the entire genome are smaller than those in healthy cells (Non-Patent Document 1). In addition, it has been reported that regarding the DNA methylation rate of the FHIT gene (Non-Patent Document 2), DLC1 gene (Non-Patent Document 3), TFPI-2 gene (Non-Patent Document 4), ABCB1 gene (Non-Patent Document 5), p16 gene (Non-Patent Document 6), and DAPK1 gene (Non-Patent Document 7), quantitative values of methylation amounts in lymphoma are different from those in healthy cells. However, there is no clear association between the DNA methylation and the prognosis for chemotherapy for lymphoma.

### [Citation List]

### [Non-Patent Document]

[Non-Patent Document 1]
   Pelham et al., Research in Veterinary Science, 2003, vol.74, p.101-104.
[Non-Patent Document 2]
   Hiraoka et al., Journal of Veterinary Medical Science, 2009, vol.71 (6) p.769-777.
[Non-Patent Document 3]
   Bryan et al., BMC Genetics, 2009, 10:73.
[Non-Patent Document 4]
   Ferraresso et al., PLOS ONE, 2014, vol.9 (4), e92707.
[Non-Patent Document 5]
   Tomiyasu et al., The Veterinary Journal, 2014, vol.199, p.103-109.
[Non-Patent Document 6]
   Fujiwara-Igarashi et al., Research in Veterinary Science, 2014, vol.97, p.60-63.
[Non-Patent Document 7]
   Sato et al., Veterinary and Comparative Oncology, 2018, vol.16, p.409-415.
[Non-Patent Document 8]
   Yamazaki et al., Veterinary Journal, 2018, vol.231, p.48-54.

### [Summary of Invention]

### [Technical Problem]

An object of the present invention is to provide a method of predicting the possibility that a post-chemotherapy prognosis for a canine with lymphoma will be favorable.

### [Solution to Problem]

As a result of diligent investigations to solve the above problems, the present inventors quantitatively analyzed a site of DNA methylation change for canine multi-central lymphoma cases, and as a result, found that in a group having a favorable post-chemotherapy prognosis and a group having a poor post-chemotherapy prognosis, two CpG sites having a significant difference in methylation rate were found, thereby completing the present invention.

That is, the present invention is as follows.
[1] A post-chemotherapy prognosis prediction method for a canine with lymphoma, which is a method for predicting a prognosis after chemotherapy for a canine with lymphoma, the prognosis prediction method including:
   a measurement step of measuring a methylation rate of one or more CpG sites selected from the group consisting of Chr4: 29559893 and Chr1: 14232692, in DNA recovered from a biological sample collected from a canine as a prediction target; and
   a prediction step of predicting the prognosis after chemotherapy for a canine with lymphoma, based on the methylation rate measured in the measurement step and a preset reference value,
   in which the reference value is a value for differentiating between a canine having a favorable post-chemotherapy prognosis and a canine having a poor post-chemotherapy prognosis, which is set for each methylation rate of each CpG site.
[2] The prognosis prediction method according to [1], in which the lymphoma is multi-central type.
[3] The prognosis prediction method according to [2], in which a sub-stage of the lymphoma is a or b.
[4] The prognosis prediction method according to any one of [1] to [3], in which the chemotherapy is CHOP therapy.
[5] The prognosis prediction method according to any one of [1] to [4], in which in the prediction step, in a case where the methylation rate of Chr4: 29559893 is equal to or less than the preset reference value, it is determined that there is a high possibility that the prognosis after chemotherapy for a canine with lymphoma is favorable.
[6] The prognosis prediction method according to [5], in which the preset reference value is 45%.
[7] The prognosis prediction method according to any one of [1] to [6], in which in the prediction step, in a case where the methylation rate of Chr1: 14232692 is equal to or less than the preset reference value, it is determined that there is a high possibility that the prognosis after chemotherapy for a canine with lymphoma is favorable.
[8] The prognosis prediction method according to [7], in which the preset reference value is 10%.
[9] The prognosis prediction method according to any one of [1] to [8], in which in the measurement step, the methylation rate of the CpG site is measured by a bisulfite pyrosequencing method.

### [Advantageous Effects of Invention]

By the post-chemotherapy prognosis prediction method for a canine with lymphoma according to the present invention, it is possible to predict a post-chemotherapy prognosis by examining a methylation rate of a specific CpG site in a genomic DNA regarding a biological sample collected from a canine with lymphoma, which is a subject animal.

### [Brief Description of Drawings]

FIG. 1 shows a diagram in which genome-wide DNA methylation analysis is performed in canine DLBCL24 cases in Example 1, methylation rates (%) in normal blood of 45 CpG sites identified as CpG sites with high methylation in the poor prognosis group are plotted on the X axis, and methylation rates (%) of the favorable prognosis group and the poor prognosis group are plotted on the Y axis.
FIG. 2A is a diagram showing a methylation rate (%) of Chr4: 29559893 obtained by bisulfite pyrosequencing in canine DLBCL14 cases in Example 2. The cases were classified into a high methylation group and a methylation group with 43% as a boundary value.
FIG. 2B is a diagram showing survival curves of a high methylation group and a low methylation group of Chr4: 29559893 in Example 2.
FIG. 2C is a diagram showing relapse-free period curves of a high methylation group and a low methylation group of Chr4: 29559893 in Example 2.
FIG. 3A is a diagram showing a methylation rate (%) of Chr4: 29559893 and Chr1: 14232692 of canine DLBCL96 cases in Example 3.
FIG. 3B is a diagram showing survival curves of a high methylation group and a low methylation group of Chr4: 29559893 and Chr1: 14232692 in Example 3.
FIG. 4A is a diagram showing survival curves of a group in which both Chr4: 29559893 and Chr1: 14232692 are low methylation groups (low methylation group 1), a group in which one of Chr4: 29559893 and Chr1: 14232692 is a low methylation group and the other is a high methylation group (low methylation group 2), and a group in which both Chr4: 29559893 and Chr1: 14232692 are high methylation groups (high methylation group), among canine DLBCL96 cases, in Example 3.
FIG. 4B is a diagram showing a proportion (%) that remitted cases and non-remitted (non-remission) cases occupy in a .total number of cases (96 cases) in the low methylation group 1 and the low methylation group 2 in Example 3.

### [Description of Embodiments]

In a cytosine base at a CpG site in genomic DNA, a carbon at the 5-position is subjected to methylation modification. In the present invention and the specification of the present application, the methylation rate of a CpG site means a proportion (%) of a methylation cytosine amount to a sum of a measured amount of methylated cytosine bases (methylation cytosine) and a measured amount of non-methylated cytosine bases (non-methylation cytosine) among the CpG site in a biological sample collected from one organism.

The post-chemotherapy prognosis prediction method for a canine with lymphoma according to the present invention (hereinafter, referred to as "prognosis prediction method according to the present invention") is a method of predicting a post-chemotherapy prognosis for a canine with lymphoma, including the following measurement step and prediction step.

A measurement step of measuring a methylation rate of one or more CpG sites selected from the group consisting of Chr4: 29559893 and Chr1: 14232692, in DNA recovered from a biological sample collected from a canine as a prediction target and,
a prediction step of predicting a post-chemotherapy prognosis for a canine with lymphoma, based on the methylation rate measured in the measurement step and a preset reference value.

The prognosis prediction method according to the present invention is a method of predicting a post-chemotherapy prognosis by specifying a DNA methylation change site that correlates with the prognosis for a multi-central lymphoma case of canine, and performing quantitative analysis on a methylation rate of the DNA methylation change site using the DNA methylation change site as a biomarker. By carrying out the method at the time of diagnosing lymphoma or before the start of chemotherapy after diagnosis, it is possible to specify a favorable prognosis group of the chemotherapy at the time of diagnosis.

In the prognosis prediction method according to the present invention, the lymphoma for which prognosis is to be predicted is not particularly limited as long as the lymphoma is a disease in which any of lymphocyte cells become tumors. Examples of the lymphoma of canine include multi-central lymphoma in which lymph nodes on a body surface are enlarged, gastrointestinal lymphoma in which the digestive organs and lymph nodes associated therewith are enlarged, thymic (mediastinal) lymphoma in which the thymus or mediastinum is enlarged, cutaneous lymphoma in which the skin or oral mucosa is enlarged, extranodal lymphoma in which a living body site other than these is enlarged, and the like. In the prognosis prediction method according to the present invention, as the lymphoma for which prognosis is to be predicted, multi-central lymphoma, which accounts for 80% of canine lymphomas, is preferable. The multi-central lymphoma mainly enlarges a mandibular lymph node, a superficial cervical lymph node, an axillary lymph node, and a hippocampal lymph node, but in the multi-central lymphoma as a prediction target in the prognosis prediction method according to the present invention, any of the lymph nodes may be enlarged. In addition, the lymphoma as a prediction target in the prognosis prediction method according to the present invention may be primary or metastatic.

In the prognosis prediction method according to the present invention, a malignant degree of the lymphoma for which the prognosis is to be predicted is not particularly limited. For example, the prognosis prediction method according to the present invention may be to predict prognosis for chemotherapy for a subject canine with lymphoma in which a sub-stage is a (state in which no clinical signs are observed), or may be to predict prognosis for chemotherapy for a subject canine with lymphoma in which a sub-stage is b (state in which clear clinical signs are observed).

In the prognosis prediction method according to the present invention, the chemotherapy as a target for prognosis prediction is not particularly limited as long as the chemotherapy is applied to a canine with lymphoma. In the present invention, it is preferable to predict prognosis for the CHOP therapy, since the chemotherapy is a standard chemotherapy for a canine with lymphoma. The CHOP therapy can be performed according to a common method (<on line>
https://www.cancer.gov/publications/dictionaries/cancer-terms/def/chop-regimen).

In the prognosis prediction method according to the present invention, the canine species of the canines as prediction targets (subject canine) is not particularly limited. Examples of the canine species of a subject canine include American Cocker Spaniel, Pembroke Welsh Corgi, Airedale Terrier, Cavalier King Charles Spaniel, Golden Retriever, Shih Tzu, German Shepherd, Jack Russell Terrier, Standard Poodle, Spitz, Chinese Crested Dog, Chihuahua, Toy Poodle, Bernese Mountain Dog, Basenji, Beagle, Bull Terrier, French Bulldog, Boxer, Border Collie, Pomeranian, Mastiff, Maltese, Miniature Schnauzer, Miniature Dachshund, Miniature Pinscher, Yorkshire Terrier, Labrador Retriever, Wire Fox Terrier, Shiba, and the like. In addition, examples may include miscellaneous species. As a subject canine in the prognosis prediction method according to the present invention, Golden Retrievers, Boxers, or the like, which are species with a high lymphoma occurrence rate, are preferable.

In the measurement step, the methylation rate of one or more CpG sites selected from the group consisting of Chr4: 29559893 and Chr1: 14232692 in DNA recovered from a biological sample collected from a subject canine is measured. That is, in the present invention, the methylation rate of at least one CpG site of Chr4: 29559893 and Chr1: 14232692 is used as a biomarker for the prognosis for chemotherapy for a canine with lymphoma. Table 2 shows a base sequence of each CpG site. In base sequences listed in the table, underlined cytosine is a target CpG site.

**[Table 2]**

| CpG site | Base sequence | SEQ NO. |
|---|---|---|
| Chr4: 29559893 | CTGGGTTCTGCGGACTTTGAGACAGGCCCCCTCTGTCTCCGAGTGGGTCGGTG GCTGTGCTCCC C GGGACTCCAGACATTCGGATCGGAATTCGCTGGTGCCTA CAAAATGCTTGCAAAAACGGTTCTCTTGCAACTAAGGAAGAGTAGA | 1 |
| Chr1: 14232692 | GCGCGGGAACGCTGCGGAGAAGTCACACGGGCTCCCGAGGACTCGCACGGACT CTCGGTGGAGCAGGCAGCCGGCCCAGGGCACCCGCCCTCGGGGCCC C GGGG GCTCCCACCGCACGCGCCAGGGGCAGGTGATCGCCTTTCCTGCGTA | 2 |

Regarding both Chr4: 29559893 and Chr1: 14232692, among CpG sites in genomic DNA, the methylation rate had a significant difference between a canine group in which the post-chemotherapy prognosis was favorable and a canine group in which the post-chemotherapy prognosis was poor, in canine with lymphoma which underwent chemotherapy.

The biological sample provided for the prognosis prediction method according to the present invention is not particularly limited as long as the sample is one collected from the subject canine and containing genomic DNA of the subject canine, and may be lymphatic fluid, blood, plasma, serum, tears, saliva, and the like, or may be a tissue sample collected from gastrointestinal mucosa or other tissues such as the liver. The biological sample provided for the prognosis prediction method according to the present invention more strongly reflects a state of lymphoma, and thus is preferably a lymphatic fluid, and more preferably a lymphatic fluid collected from an enlarged lymph node. In addition, from the viewpoint of being minimally invasive and suppressing the burden on the subject canine, blood, plasma, serum, tears, saliva, and the like are also preferable. In a case where a biological sample such as a body fluid such as the lymphatic fluid or a tissue is collected, the biological sample may be collected using a collecting tool corresponding to each biological sample.

In addition, the biological sample may be in a state in which DNA can be extracted, or may be subjected to various pre-treatments. For example, the sample may be a formalin-fixed paraffin-embedded (FFPE) tissue. DNA can be extracted from the biological sample by a common method, and various commercially available DNA extraction! purification kits can also be used.

The method of measuring a methylation rate of a CpG site is not particularly limited as long as the method is capable of distinguishing and quantifying a methylated cytosine base and an unmethylated cytosine base for a specific CpG site. The methylation rate of the CpG site can be measured by a known method as it is or by appropriately modifying a known method depending on the necessity in the technical field. Examples of the method of measuring a methylation rate of a CpG site include a bisulfite sequencing method, a methylation-specific polymerase chain reaction (MSP), and the like.

In the prognosis prediction method according to the present invention, as the prediction step, a post-chemotherapy prognosis for the canine with lymphoma is predicted based on a methylation rate measured in the measurement step and a preset reference value. The reference value is a value for differentiating between a canine for which a post- chemotherapy prognosis is favorable and a canine for which a post-chemotherapy prognosis is poor, which is set for each methylation rate of each CpG site of Chr4: 29559893 and Chr1: 14232692. Canines of which a methylation rate at each CpG site is equal to or less than the reference value may be referred to as a low methylation group, and canines of which a methylation rate at each CpG site is more than the reference value may be referred to as a high methylation group.

In the prognosis prediction method according to the present invention, in the prediction step, in a case where the methylation rate of Chr4: 29559893 is equal to or less than a preset reference value, the subject canine is in a low methylation group and it is determined that there is a high possibility that the prognosis after the subject canine undergoes chemotherapy is favorable. Similarly, in the prediction step, in a case where the methylation rate of Chr1: 14232692 is equal to or less than the preset reference value, the subject canine is in the low methylation group, and it is determined that there is a high possibility that the prognosis after the subject canine undergoes chemotherapy is favorable.

The reference value of the methylation rate at each CpG site can be, for example, experimentally obtained as a threshold value with which a methylation rate at the CpG site, which is used as a biomarker, of a group that has experienced remission at least once and favorable prognosis and a methylation rate at the CpG site of a group that has never experienced remission and poor prognosis, among the canines with lymphoma subjected to chemotherapy, are compared with each other and both groups can be distinguished from each other.

For example, the reference value of the methylation rate of Chr4: 29559893 can be set within a range of 25% to 55%. For example, in a case where the reference value is set to 45%, and in a case where the methylation rate of Chr4: 29559893 of the subject canine is 45% or less, it is determined that there is a high possibility that the prognosis is favorable in a case where the subject canine undergoes chemotherapy. In a case where the methylation rate of Chr4: 29559893 of the subject canine is more than 45%, it is determined that there is a high possibility that the prognosis is not favorable in a case where the subject canine undergoes chemotherapy.

For example, the reference value of the methylation rate of Chr1: 14232692 can be set within a range of 5% to 30%. For example, in a case where the reference value is set to 10%, and in a case where the methylation rate of Chr1: 14232692 of the subject canine is 10% or less, it is determined that there is a high possibility that the prognosis is favorable in a case where the subject canine undergoes chemotherapy. In a case where the methylation rate of Chr1: 14232692 of the subject canine is more than 10%, it is determined that there is a high possibility that the prognosis is not favorable in a case where the subject canine undergoes chemotherapy.

In the prediction step of the prognosis prediction method according to the present invention, the prognosis of chemotherapy of a subject canine may be predicted only from the methylation rate of Chr4: 29559893, or may be predicted only from the methylation rate of Chr1: 14232692, but a more reliable prediction can be performed in a case where the prediction is performed based on both the methylation rate of Chr4: 29559893 and the methylation rate of Chr1: 14232692. For example, in a case where the methylation rate of Chr4: 29559893 is more than the reference value and the methylation rate of Chr1: 14232692 is more than the reference value, it is predicted that the prognosis is poor even if chemotherapy is performed on the subject canine. In addition, in a case where the methylation rate of Chr4: 29559893 is equal to or less than the reference value and the methylation rate of Chr1: 14232692 is equal to or less than the reference value, it is predicted that the prognosis is favorable even if chemotherapy is performed on the subject canine.

The prognosis prediction method according to the present invention can provide important information for supporting decision on whether or not a veterinarian performs chemotherapy on a canine with lymphoma. Therefore, it is preferable that the prognosis prediction method according to the present invention be performed before performing chemotherapy on a canine with lymphoma. It is possible to further enhance the treatment efficiency by performing chemotherapy on subject canines predicted to have favorable prognosis by the prognosis prediction method according to the present invention, and by not performing chemotherapy on subject canines predicted to have poor prognosis.

### [Examples]

The present invention will be described in more detail below with reference to examples, but the present invention is not limited thereto.

### [Example 1]

By Canine DREAM (Non-Patent Document 8), which is a genome-wide analysis method for DNA methylation established by the inventors of the present invention, comprehensive analysis of a large number (about 100,000) of CpG sequences in a canine genome including not only the promotor region but also CpG sites of exon or intron in the gene and other portions in the gene was performed on 24 cases of canine multi-central lymphoma, and two CpG sequences capable of differentiating with a favorable prognosis group and a poor prognosis group were specified (Test cohort).

### (1) Subject canine

Twenty-four cases of spontaneous onset canine multi-central high-malignant lymphoma (MHGL) were used as subject canines. MHGL was diagnosed by a pathologist based on cytological findings (high dividing rate and medium to large cells), and grading was performed by the WHO livestock lymphoma clinical grading system. All cases were treated with a minimum of one CHOP anticancer drug treatment.

### (2) Extraction of genomic DNA

Genomic DNA was extracted from a cytology test slide of each case using a DNA extraction kit "DNeasy Blood & Tissue Kit" (manufactured by Qiagen).

### (3) Canine DREAM method

Genome-wide DNA methylation was analyzed by next-generation sequencing. Genomic DNA (2 µg) extracted from a sample was mixed with 2 pg of an artificial calibrator (methylation levels of 0, 25, 50, 75, and 100%). The obtained mixture was cleaved by treating with 100 U of SmaI enzyme at 25°C for 3 hours, and then reacted with 50 U of XmaI enzyme at 37°C for 16 hours. The cleaved DNA was purified with magnetic beads "Agencourt AMPure XP" (manufactured by Beckman Coulter, Inc.). 0.4 mM of dCTP, dGTP, and dATP were added to the 3'-protruding end of the XmaI-cleaved DNA, and a 3'dA terminal was added to a control fragment with Klenow DNA polymerase deficient in 3'-5' exonuclease activity (manufactured by New England Biolabs, Inc.).

Thereafter, the Illumina paired-end sequencing adapter was bonded with T4 DNA ligase (manufactured by New England Biolabs, Inc.). For the size of the ligation mix, dual-SPRI size selection was performed using Agencourt AMPure XP, and a DNA fragment of 250 to 450 base pairs (bp) was extracted. The purified DNA was amplified in 11 cycles with an Illumina pair-end PCR primer and KAPA Hifi Hotstart Ready Mix (Kapa Biosystems, Inc.). The amplified sequence library was purified with the magnetic beads, and sequenced with Illumina HiSeq 2000 (manufactured by Illumina, Inc.). The sequenced reads were mapped to the SmaI/XmaI site of the canine genome (canFam3.1), and a signature of methylated and non-methylated CpG at the SmaI/XmaI site was calculated. Thereafter, a methylation frequency of the single SmaI/XmaI site was calculated. A methyl ratio was calculated by dividing the number of tags starting from CCGGG by the total number of tags mapped to the SmaI/XmaI site.

Methylation levels calculated by DREAM were corrected based on standard spikes. Log ratios ln (m/u) and ln (sm/u) were calculated for each standard. m/u is an expected methylation ratio to a non-methylation ratio, and sm and u each are the observed methylation ratio and non-methylation ratio. The log ratio of "observation" was subtracted from "prediction" for each standard to calculate. The calculation of correction coefficient (c) was performed by an average antilog of log([prediction] - [observation]), and the corrected methyl values for the individual CpG sites were calculated to 100% × [c × sm/(c × [sm + u])].

The methylation level of a single SmaI/XmaI site was analyzed using a minimum of 20 sequencing reads.

### (4) Statistical analysis

In the genome-wide DNA methylation analysis, DNA methyl levels between groups were analyzed by t-test. For correction of multiple tests, methylation differences were confirmed at 10% FDR using the Benjamini-Hochberg method. A survival curve of Test cohort was drawn by the Kaplan-Meier method. In MHGL cases, average survival periods of case groups having different DNA methylation patterns at CpG sites were compared. Significant differences between the survival curves were compared by the log rank test. Data for mortal cases not related to MHGL, traceable cases, or living cases were cleaved and excluded.

As a control, the CpG methylation rate was also measured in the same manner for the genomic DNA derived from three kinds of normal peripheral blood.

### (6) Results

In the present experiment, 40,128 CpG sites for which data were obtained in common in all samples of 24 cases were used for analysis. Clustering analysis was performed using 16,992 CpG sites in CpG islands, and it was found that cases were divided into a plurality of groups.

In a case where survival curves and remission periods were drawn for the plurality of groups, significant differences were obtained in each group. From these results, it was found that a favorable prognosis group for chemotherapy was extracted by the genome-wide DNA methylation pattern of CpG islands.

Subsequently, to search for the CpG site that defines the favorable prognosis group, a volcano plot analysis was performed to directly compare the favorable prognosis group and the poor prognosis group. In the volcano plot analysis, to increase the detection power, average values of 9 cases of the favorable prognosis group and 15 cases of the other poor prognosis group at the 38,693 sites of CpG islands, where the comparative analysis was possible in common in 20 or more cases, which were 80% or more of the 24 cases, were obtained, and the difference and p value are shown.

Regarding the number of CpG sites showing a difference of methylation of 20% or more and a statistically significant difference of 0.01 or less, 520 CpG sites with high methylation in the poor prognosis group and 414 CpG sites with high methylation in the favorable prognosis group, that is, a total of 934 CpG sites were identified. Among these, as a result of calculating the number of related genes from CpG sites considered to be sites in which genes are in the vicinity and which are present in the promoter region, it was found that CpG sites with high methylation in the poor prognosis group were in 45 genes, while CpG sites showing opposite tendencies were in 5 genes, and many genes exhibited high methylation in the poor prognosis group.

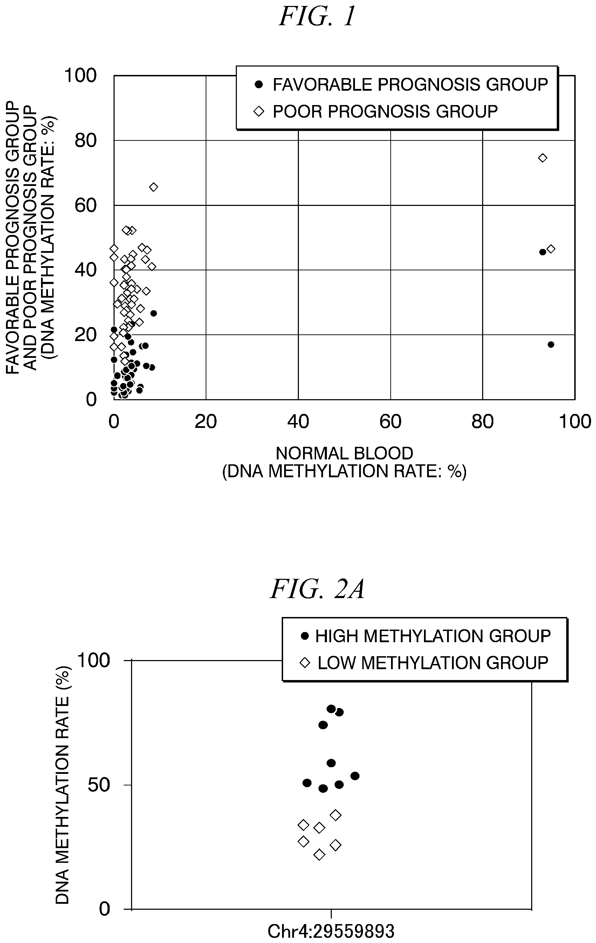

Subsequently, analysis on the 45 genes and 45 CpG sites was performed. FIG. 1 is a diagram in which methylation rates (%) of these 45 CpG sites in normal blood are plotted on the X axis, and methylation rates (%) in the favorable prognosis group and the poor prognosis group are plotted on the Y axis. As shown in FIG. 1, the methylation rate of most of these CpG sites was as low as 20% or less in normal blood, and the methylation rate of most of these CpG sites was 20% or less even in the favorable prognosis group, but only the poor prognosis group exhibited more than 20% of the methylation rate and some exhibited the methylation rate of 40% to 60%. In addition, by gene ontology analysis, it was found that there were many genes important for signal transmission such as BMP and WNT and differentiation and maturation, among these 45 genes (Table 3).

**[Table 3]**

| Name | Number | Gene | Benjamini |
|---|---|---|---|
| Signal | 18 | FGF5, UCN, GRIK3, DUOX1, TRIL, GDNF, DKK2, ST6GALNAC5, NPY, SCG3, PLA2G7, GFRA1, FBN2, BMP7, COL24A1, ADGRA2, VLDLR, NXPH1 | 0.001122 |
| Secreted | 8 | WNT5A, FGF5, NPY,PLA2G7, COL24A1, BMP7, WNT7A, GDNF | 0.001749 |
| Developmental protein | 5 | WNT5A, TBX2, NEUROD2, BMP7, WNT7A | 0.004707 |

### [Example 2]

It was examined whether or not a prognostic state in a new case group can be predicted by DNA methylation analysis of candidate CpG sites identified as prognostic markers for chemotherapy in Example 1. Specifically, 14 new DLBCL cases different from those in Example 1 and cases were analyzed using bisulfite pyrosequencing, which has high reliability related to local analysis, is inexpensive, and swift compared to DREAM, and enabled simple analysis this time. As the target CpG site, a top-ranked CpG site (Chr4: 29559893) was selected by the DREAM analysis in Example 1, together with the difference and significant difference in methylation rates between the favorable prognosis group and the poor prognosis group.

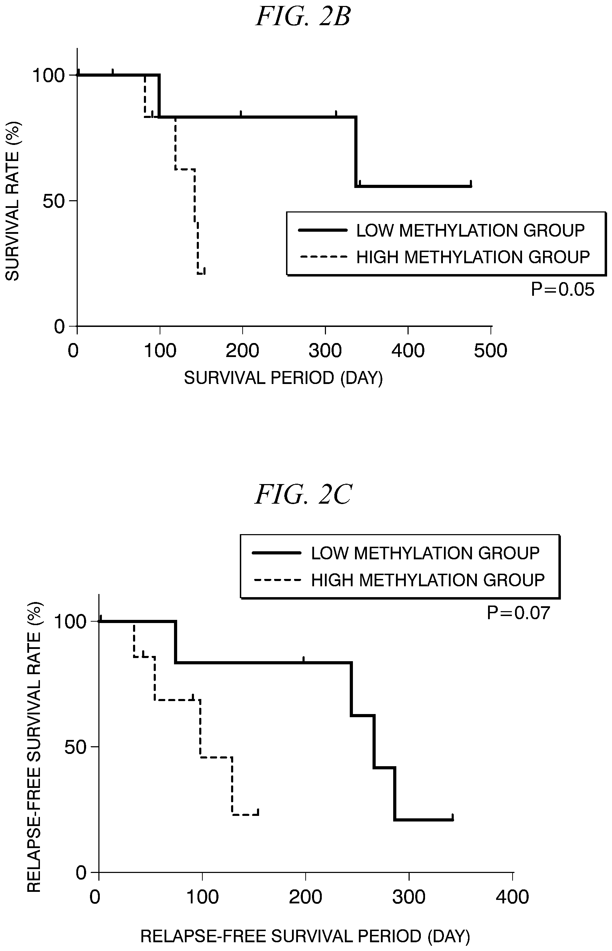

FIG. 2A shows the methylation rates (%) obtained by the bisulfite pyrosequencing of Chr4: 29559893 of 14 cases. As a result of classifying the 14 cases this time, using 43% as a boundary value, which is the average methylation rate + SD value of this CpG site in the favorable prognosis group in the DREAM analysis, the cases were classified into 8 cases of the high methylation group and 6 cases of the low methylation group. Subsequently, for the high methylation group and the low methylation group, survival curves were drawn in FIG. 2B and relapse-free period curves were drawn in FIG. 2C. As a result, it was re-confirmed that both the survival period and the relapse-free period correlate with low methylation in the favorable prognosis group, and it was confirmed that the CpG site related to prognosis can be identified by the DREAM analysis.

### [Example 3]

For Chr4: 29559893 and Chr1: 14232692, which are two top-ranked CpG sites in terms of difference and significant difference in methylation rates of the favorable prognosis group and the poor prognosis group, specified from the genome-wide DNA methylation analysis by the canine DREAM method of Example 1, as a confirmation experiment, DNA was extracted from 96 cases of cell sample slides diagnosed with canine multi-central lymphoma, and the DNA methylation level was quantified using the bisulfite pyrosequencing method (bisulfite treatment and pyrosequencing method) for the CpG sequences (Validation cohort).

### (1) Subject canine

96 cases of MHGL different from Example 1 were used as subject canines. MHGL was diagnosed by a pathologist based on cytological findings (high dividing rate and medium to large cells), and grading was performed by the WHO livestock lymphoma clinical grading system. All cases were treated with a minimum of one CHOP anticancer drug treatment.

Among the 96 cases, a sub-stage a (state in which no clinical signs were observed) was 67 cases (70%), and a sub-stage b (state in which clear clinical signs were observed) was 29 cases (30%). In addition, canine species of the 96 cases of subject canines are shown in Table 4.

**[Table 4]**

| Canine species name | % |
|---|---|
| American Cocker Spaniel | 2.1 |
| Pembroke Welsh Corgi | 12.5 |
| Airedale Terrier | 1.0 |
| Cavalier King Charles Spaniel | 5.2 |
| Golden Retriever | 4.2 |
| Miscellaneous Species | 4.2 |
| Shih Tzu | 8.3 |
| German Shepherd | 1.0 |
| Jack Russell Terrier | 3.1 |
| Standard Poodle | 2.1 |
| Spitz | 2.1 |
| Chinese Crested Dog | 1.0 |
| Chihuahua | 6.3 |
| Toy Poodle | 2.1 |
| Bernese Mountain Dog | 2.1 |
| Basenji | 1.0 |
| Beagle | 3.1 |
| Bull Terrier | 2.1 |
| French Bulldog | 3.1 |
| Border Collie | 1.0 |
| Pomeranian | 1.0 |
| Mastiff | 1.0 |
| Maltese | 1.0 |
| Miniature Schnauzer | 1.0 |
| Miniature Dachshund | 13.5 |
| Miniature Pinscher | 1.0 |
| Yorkshire Terrier | 3.1 |
| Labrador Retriever | 2.1 |
| Wire Fox Terrier | 1.0 |
| Shiba | 7.3 |
| Total | 100 |

### (2) Extraction of genomic DNA

Extraction of genomic DNA from the cytological examination slide of each case was performed in the same manner as in Example 1.

### (3) Bisulfite treatment and pyrosequencing

The DNA methylation rate at the specified target CpG site (Chr4: 29559893 and Chr1: 14232692) was quantified and evaluated by the bisulfite pyrosequencing method. The genomic DNA was subjected to a bisulfite treatment using "EZ DNA Methylation-Lighting Kit" (manufactured by Zymo Research Corporation). Subsequently, a region including the CpG site was amplified by two-step PCR using a primer specific to the target CpG site. Regarding the DNA methylation rate at CpG sites, a percentage of the DNA methylation level (CpG methylation rate) was measured by pyrosequencing using Pyro-Gold Reagent Kit (manufactured by QIAGEN N. V.) of a PSQ24 system.

### (4) Statistical analysis

In the genome-wide DNA methylation analysis, DNA methyl levels between groups were analyzed by t-test. For correction of multiple tests, methylation differences were confirmed at 10% FDR using the Benjamini-Hochberg method. The survival curve of the validation cohort was drawn by the Kaplan-Meier method. In the MHGL cases of the validation cohort, the average survival period of case groups having different DNA methylation patterns at the specified target CpG site was compared. Significant differences between the survival curves were compared by the log rank test. Data for mortal cases not related to MHGL, traceable cases, or living cases were cleaved and excluded.

### (5) Results

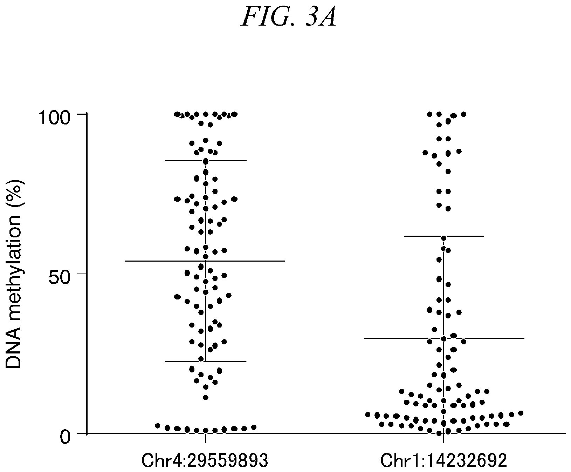

FIG. 3A shows methylation rates (%) of Chr4: 29559893 and Chr1: 14232692 of 96 cases. As shown in FIG. 3A, DNA methylation levels showed heterogeneity at both CpG sites (various DNA methylation levels are shown depending on the case). In addition, both CpG sites also had a success rate of more than 97% related to the measurement of DNA methylation levels.

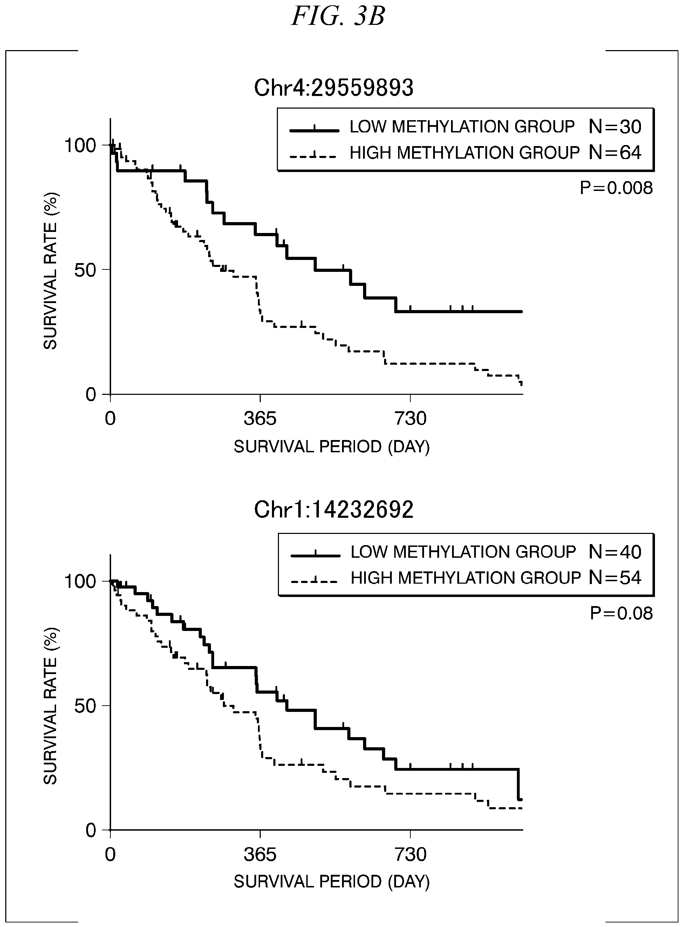

From the results in FIG 3A, for convenience, the reference values for classifying the high methylation group and the low methylation group were set to 45% for Chr4: 29559893 and 10% for Chr1: 14232692, and regarding the high methylation group and the low methylation group, the survival curves were drawn in FIG. 3B. As a result, it was shown that for both CpG sites, the prognosis of the high methylation group was poorer than that of the low methylation group, in other words, the prognosis of the low methylation group was better. These results were consistent with the results in Examples 1 and 2.

In addition, using the results of FIG. 3A, the methylation rates of both Chr4: 29559893 and Chr1: 14232692 were combined to predict prognosis. Among canine DLBCL96 cases, a group in which both Chr4: 29559893 and Chr1: 14232692 were the low methylation groups (low methylation group 1) had 17 cases, a group in which any one of Chr4: 29559893 or Chr1: 14232692 was the low methylation group and the other was the high methylation group (low methylation group 2) had 74 cases, and a group in which both Chr4: 29559893 and Chr1: 14232692 were the high methylation groups (high methylation group) had 31 cases. In the group in which at least one of Chr4: 29559893 and Chr1: 14232692 was the low methylated group, the low methylation group 1 in which both were the low methylated groups was 18%, and the low methylation group 2 in which any one was the low methylation group was 82%.

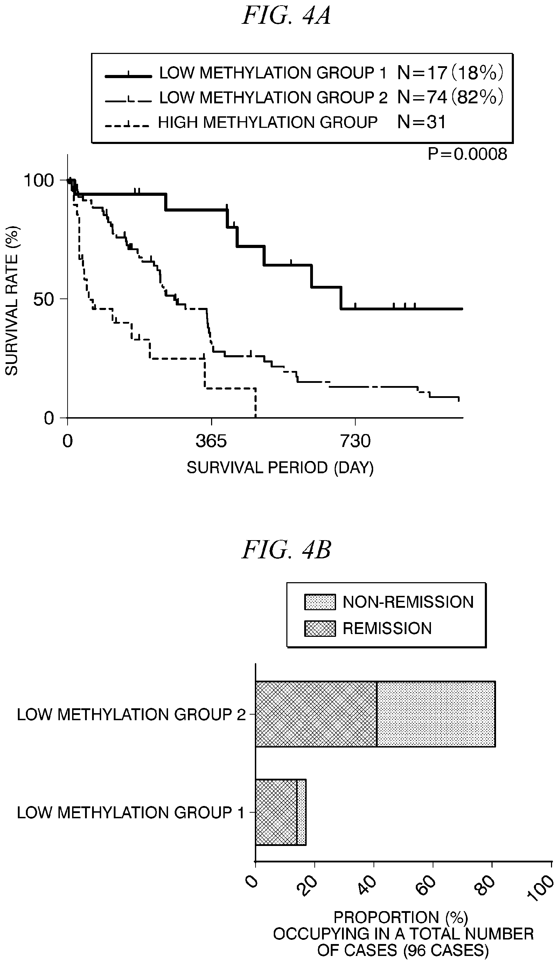

FIG. 4A shows survival curves of the low methylation group 1, the low methylation group 2, and the high methylation group. The low methylation group 1 showed significantly favorable prognosis as compared with the low methylation group 2 or the high methylation group. The 2-year survival rate of the high methylation group 1 was 50%, and a significantly better prognosis was expected than the current standard prognosis.

FIG. 4B shows a proportion (%) that remitted cases and non-remitted (non-remission) cases occupied in a total number of cases (96 cases) in the low methylation group 1 and the low methylation group 2. As a result, in the low methylation group 1, 14 cases (15%) out of 17 cases (18%) had remission, and a high remission rate of 82% was shown.

These results suggest the usefulness as a biomarker for risk evaluation, prognosis determination, prediction of treatment responsiveness, and the like, which can be easily applied clinically. At this time, it was possible to apply the DNA methylation analysis of a specific region to a large number of case samples by using the pyrosequencing method with the highest reliability in the DNA methylation analysis of each individual site. For this reason, it is possible to examine disease-specific DNA methylation candidate regions at a low cost, and to mainly verify the universality for a large number of cases.

## Claims

1. A post-chemotherapy prognosis prediction method for a canine with lymphoma, which is a method for predicting a prognosis after chemotherapy for a canine with lymphoma, the prognosis prediction method comprising:
a measurement step of measuring a methylation rate of one or more CpG sites selected from the group consisting of Chr4: 29559893 and Chr1: 14232692, in DNA recovered from a biological sample collected from a canine as a prediction target; and
a prediction step of predicting the prognosis after chemotherapy for a canine with lymphoma, based on the methylation rate measured in the measurement step and a preset reference value,
wherein the reference value is a value for differentiating between a canine having a favorable post-chemotherapy prognosis and a canine having a poor post-chemotherapy prognosis, which is set for each methylation rate of each CpG site.

2. The prognosis prediction method according to Claim 1,
wherein the lymphoma is multi-central type.

3. The prognosis prediction method according to Claim 2,
wherein a sub-stage of the lymphoma is a or b.

4. The prognosis prediction method according to Claim 1,
wherein the chemotherapy is CHOP therapy.

5. The prognosis prediction method according to Claim 1,
wherein in the prediction step, in a case where the methylation rate of Chr4:
29559893 is equal to or less than the preset reference value, it is determined that there is a high possibility that the prognosis after chemotherapy for a canine with lymphoma is favorable.

6. The prognosis prediction method according to Claim 5,
wherein the preset reference value is 45%.

7. The prognosis prediction method according to Claim 1,
wherein in the prediction step, in a case where the methylation rate of Chr1:
14232692 is equal to or less than the preset reference value, it is determined that there is a high possibility that the prognosis after chemotherapy for a canine with lymphoma is favorable.

8. The prognosis prediction method according to Claim 7,
wherein the preset reference value is 10%.

9. The prognosis prediction method according to Claim 1,
wherein in the measurement step, the methylation rate of the CpG site is measured by a bisulfite pyrosequencing method.
